# EUROPEAN PATENT APPLICATION

(11) **EP 4 706 566 A1**
(43) Date of publication of application: **11.03.2026**
(21) Application number: 25811145.9
(22) Date of filing: 24.04.2025
(51) Int. Cl.: A61B 17/92, A61F 2/46

(54) **ORTHOPEDIC ADAPTER**

(30) Priority: 02.07.2024 KR 20240086654; 24.07.2024 KR 20240098216
(71) Applicant: IMEDICOM Co., Ltd., Gunpo-Si, Gyeonggi-do 15807 (KR)
(72) Inventor: LEE, Byoung Ju, Gunpo-si, Gyeonggi-do 15807 (KR)
(74) Representative: Manitz Finsterwald Patent- und Rechtsanwaltspartnerschaft mbB
(86) International application number: PCT/KR2025/005583
(87) International publication number: WO 2026/010099

(57) **Abstract**

The orthopedic adapter of the present invention is detachably coupled to a rotary electric tool, and includes a tool coupling part receiving rotational force from the rotary electric tool, a shaft rotating in interlock with a rotation direction of the tool coupling part, a first striking-part transfer part rotating in interlock with the shaft only when the shaft rotates in a first direction, a first striking part capable of moving rearward by rotation of the first striking-part transfer part, a second striking-part transfer part rotating in interlock with the shaft only when the shaft rotates in a second direction, a second striking part capable of moving forward by rotation of the second striking-part transfer part, a force transmission part capable of moving rearward by contact with the first striking part and capable of moving forward by contact with the second striking part, and a force adjustment part capable of adjusting a force that the second striking part applies to the force transmission part by adjusting acceleration force by which the second striking part moves forward.

## Description

### Technical Field

The present invention relates to an orthopedic adapter, and specifically is an adapter for an impactor used in orthopedic surgery.

### Background Art

As the aging phenomenon of the population is deepening worldwide, demand for hip replacement surgery is continuously increasing. Also, recently, even in young patients, cases of receiving hip surgery due to various inflammations or tumors, strenuous exercise, etc. are increasing.

Artificial hip replacement surgery is a surgery of replacing one side or both sides of a joint with an artificial joint when an abnormality occurs in a joint or bone connecting the hip bone (acetabulum) and the thigh bone (femur).

Meanwhile, in such a surgical process, a process of inserting a stem (Femoral Stem) into the inside of the femur (Femur) at a correct position is one of elements that determine the prognosis of the surgery. Conventionally, in order to insert the stem into the femur, first, a broach is inserted into the femur and then removed to form a space, and then the stem is inserted into this space.

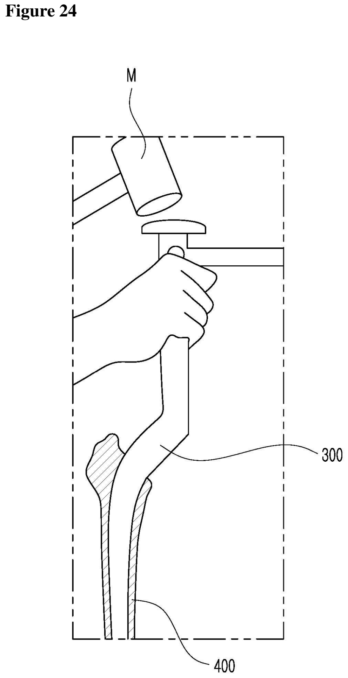

However, as shown in Fig. 24, a method was used in which a doctor directly manually applies an impact to the broach (300) using a tool such as a mallet (M) to insert it into the femur (400) and form a space for stem insertion. However, in this case, the size and direction, etc. of the impact applied to the broach (300) are not constant, so an abnormal space is formed in the femur. As a result, when inserting the stem into this space, the prognosis of the surgery becomes poor, causing a problem of leading to reoperation.

In order to improve this point, the applicant has developed an orthopedic impactor disclosed in Korean Registered Patent No. 2,343,886 (see Fig. 25 and Fig. 26). For reference, Fig. 25 and Fig. 26 correspond to Fig. 1 and Fig. 4 of the Korean registered patent owned by the applicant, respectively, and for convenience of explanation, the reference numerals used in the Korean registered patent were used as they are without modification, and it is clarified that even if these reference numerals overlap with the reference numerals used in the description of the present invention described later, they do not refer to the same components.

Such a conventional impactor includes a rotary electric tool (200) and an adapter (100) detachably coupled thereto. As shown in Fig. 24, the adapter (100) includes a case part (6), a tool coupling part (5) that receives rotational force from the rotary electric tool, a first rotating part (7) that rotates in interlock with rotation of the tool coupling part only in one direction among rotation directions of the tool coupling part, a striking-part transfer part (4) that rotates in interlock with rotation of the first rotating part, a striking part (3) that moves in a first direction while compressing a first spring (2) by rotation of the striking-part transfer part and then moves in a second direction opposite to the first direction by restoring force of the first spring, and a force transmission part (1) that moves in the second direction by contact with the striking part.

However, the conventional adapter can apply a force for striking a broach for broach insertion, but can only strike the broach only at the time of broach insertion, and had a limitation that it cannot be used for removing the broach. Also, since the conventional adapter cannot adjust the force applied to the broach, there was a problem that it could cause femur breakage due to excessive force.

### Detailed Description of the Invention

### Technical Problem

The present invention is for solving the problems as described above, and an object of the present invention is to provide an orthopedic adapter that can adjust striking force for insertion of a surgical instrument such as a broach, and also can generate not only striking force for insertion of the surgical instrument but also a force for removing the surgical instrument.

### Technical Solution

In order to achieve the above object, an orthopedic adapter according to one embodiment of the present invention is detachably coupled to a rotary electric tool, and is characterized by including a case part, a tool coupling part receiving rotational force from the rotary electric tool, a shaft rotating in interlock with a rotation direction of the tool coupling part, a first striking-part transfer part rotating in interlock with the shaft only when the shaft rotates in a first direction, a first striking part capable of moving rearward by rotation of the first striking-part transfer part, a second striking-part transfer part rotating in interlock with the shaft only when the shaft rotates in a second direction, a second striking part capable of moving forward by rotation of the second striking-part transfer part, a force transmission part capable of moving rearward by contact with the first striking part and capable of moving forward by contact with the second striking part, and a force adjustment part capable of adjusting a force that the second striking part applies to the force transmission part.

Also, the force adjustment part is characterized by including an outer ring mounted on an outside of the case part to be rotatable relative to the case part, an inner ring located inside the case part and rotating in interlock with rotation of the outer ring, a connection pin connecting the outer ring and the inner ring to each other, and a second inner case fixed to the case part between the outer ring and the inner ring inside the case part, and having a multi-stage pin hooking hole through which the connection pin passes, in which a plurality of hooking grooves in which the connection pin can be selectively caught are formed, and which is formed to be inclined to a central axis of the shaft.

Also, the second striking-part transfer part is characterized by including a second elastic member of which one end is supported by the inner ring and the other end is supported by the second striking part, and a second body portion having a second through hole through which the shaft passes, a plurality of second thread portions, a second thread valley portion formed between the plurality of second thread portions, and a second longitudinal groove portion extending in a longitudinal direction.

Also, the first striking-part transfer part is characterized by including a first elastic member of which one end is supported by a first spring support member fixed to the case part and the other end is supported by the first striking part, and a first body portion having a first through hole through which the shaft passes, a plurality of first thread portions, a first thread valley portion formed between the plurality of first thread portions, and a first longitudinal groove portion extending in a longitudinal direction.

Also, the shaft has second shaft thread portions and second shaft valley portions alternately positioned in a circumferential direction thereof, the second body portion has second ratchet parts, the second ratchet parts have a second hook part and a second pressing part applying elastic force in a direction of always pressing the second hook part, and the second hook part can be caught in the second shaft valley portion and, when the shaft rotates in the second direction, does not disengage from the second shaft valley portion so that the second body portion can rotate in interlock with the shaft.

Also, when the shaft rotates in the first direction, the second hook part overcomes elastic force of the second pressing part by rotational force of the shaft and moves sequentially to another neighboring second shaft valley portion while rotating relative to the shaft, whereby the second body portion is characterized in that it does not interlock with rotation of the shaft.

Also, the shaft further has first shaft thread portions and second shaft valley portions alternately positioned in the circumferential direction thereof, the first body portion has first ratchet parts, the first ratchet parts have a first hook part and a first pressing part applying elastic force in a direction of always pressing the first hook part, and the first hook part can be caught in the first shaft valley portion and, when the shaft rotates in the first direction, does not disengage from the first shaft valley portion so that the first body portion can rotate in interlock with the shaft.

Also, when the shaft rotates in the second direction, the first hook part overcomes elastic force of the first pressing part by rotational force of the shaft and moves sequentially to another neighboring first shaft valley portion while rotating relative to the shaft, whereby the first body portion is characterized in that it does not interlock with rotation of the shaft.

Also, the first striking part includes a first body part in which a first bore is formed, a first bar penetrating a wall of the first body part so that one end protrudes into the first bore and the other end protrudes to an outside of the first body part, and a first protrusion part connected to one end of the first body part to form a step with the first body part, and is characterized in that two first bars are provided to face each other at 180° intervals in a circumferential direction of the first body part.

Also, one end of the first bar moves along the first thread valley portion when the first body portion rotates, and when the one end of the first bar leaves the first thread valley portion and reaches the first longitudinal groove portion, the first striking part is characterized in that it moves rearward by restoring force of the first elastic member.

Also, the second striking part includes a second body part in which a second bore is formed, a second bar penetrating a wall of the second body part so that one end protrudes into the second bore and the other end protrudes to an outside of the second body part, and a second protrusion part connected to one end of the second body part to form a step with the second body part, and is characterized in that two second bars are provided to face each other at 180° intervals in a circumferential direction of the second body part.

Also, one end of the second bar moves along the second thread valley portion when the second body portion rotates, and when the one end of the second bar leaves the second thread valley portion and reaches the second longitudinal groove portion, the second striking part is characterized in that it moves forward by restoring force of the second elastic member.

Also, the case part is characterized by further including a circumferential through hole through which the connection pin can move, and a front opening from which the force transmission part protrudes.

Also, the force transmission part is characterized by including a tool connection part protruding through the front opening of the case part, and a frame relatively slidable along the shaft.

Also, the second inner case is characterized by further having a second slit through which the second bar moves.

Also, a first inner case fixed inside the case part and surrounding the first striking part and the first striking-part transfer part is provided, and the first inner case is characterized by having a first slit through which the first bar moves.

### Effects of the Invention

The orthopedic adapter according to an embodiment of the present invention having the above-described configuration has the following effects.

First, the present invention can adjust the force that the second striking part applies to the force transmission part by using the force adjustment part, and thus can adjust a striking force when inserting a surgical instrument into a bone; therefore, it is possible to improve the problems of a conventional impactor adapter in which bone fracture, for example, fracture of the femur, may occur due to excessive force.

Also, since it can generate not only a striking force for inserting a surgical instrument such as a broach but also a force for removing the surgical instrument, the surgical instrument can be safely and easily removed with a constant force when removing the surgical instrument.

Meanwhile, the present invention also of course includes other effects that can be expected from the above-described configuration, although not explicitly described.

### Brief Description of the Drawings

Fig. 1 is a perspective view of an orthopedic adapter according to one embodiment of the present invention.
Fig. 2 is a longitudinal cross-sectional view of the adapter of Fig. 1.

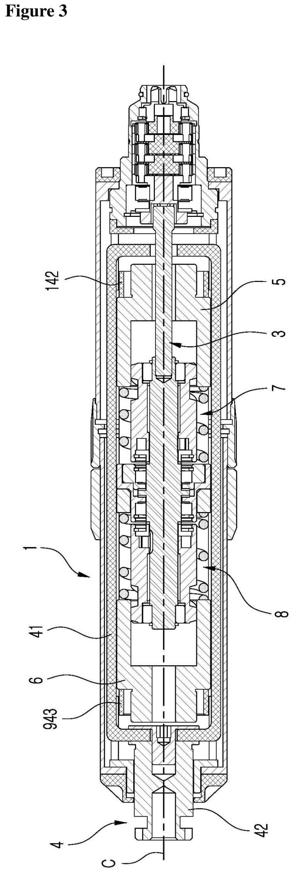

Fig. 3 is another longitudinal cross-sectional view of the adapter of Fig. 1.
Fig. 4 shows the case part of the adapter of Fig. 1.
Fig. 5 is a plan view of the adapter of Fig. 1 in a state where an outer ring is removed.
Fig. 6 (a) is a perspective view of a second inner case of the adapter of Fig. 1, and Fig. 6 (b) is a perspective view seen from another direction.
Fig. 7 shows a first inner case of the adapter of Fig. 1.
Fig. 8 shows a coupling relationship between a second striking part and a second body part in the adapter of Fig. 1.
Fig. 9 shows a coupling relationship between a first striking part and a first body part in the adapter of Fig. 1.
Fig. 10 is a schematic diagram for explaining a manner in which a force adjustment part operates in the adapter of Fig. 1.
Fig. 11 (a) shows a case where a connection pin is located in a lowest hooking groove in Fig. 10, and Fig. 11 (b) shows a case where the connection pin is located in a highest hooking groove.
Fig. 12 shows a shaft of the adapter of Fig. 1.
Fig. 13 (a) is a cross-sectional perspective view of the shaft seen in a B-B direction of Fig. 12,
Fig. 13 (b) shows a coupling relationship between the shaft and the second body part seen in an E-E direction of Fig. 2, and Fig. 13 (c) is an exploded view of main components of the second body part.
Fig. 14 (a) is a cross-sectional perspective view of the shaft seen in an A-A direction of Fig. 12, Fig. 14 (b) shows a coupling relationship between the shaft and the first body part seen in a D-D direction of Fig. 2, and Fig. 14 (c) is an exploded view of main components of the first body part.
Fig. 15 is a schematic diagram showing a coupling relationship of a force transmission part, a second striking part, a second striking-part transfer part, a first striking-part transfer part, and a first striking part in the adapter of Fig. 1.
Fig. 16 (a) shows a state in which an outer ring, an inner ring, and a connection pin of a force adjustment part of the adapter of Fig. 1 are coupled, and Fig. 16 (b) shows a state in which these are disassembled.

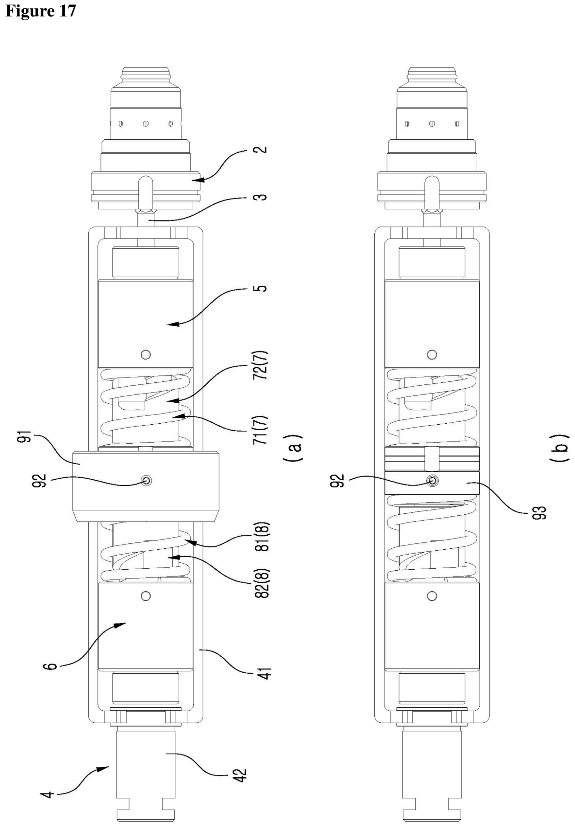

Fig. 17 (a) is a plan view in which the connection pin is shown in Fig. 15, and Fig. 17 (b) is a plan view in which the outer ring is shown.
Fig. 18 (a) to Fig. 18 (c) are views showing an action of the second striking part of the adapter of Fig. 1.
Fig. 19 (a) to Fig. 19 (c) are views respectively showing relative positions of a second bar with respect to the second body part in steps Fig. 18 (a) to Fig. 18 (c).
Fig. 20 (a) to Fig. 20 (c) are views showing an action of the first striking part of the adapter of Fig. 1.
Fig. 21 (a) to Fig. 21 (c) are views respectively showing relative positions of a first bar with respect to the first body part in steps Fig. 20 (a) to Fig. 20 (c).
Fig. 22 (a) shows a coupling relationship between the second inner case and the second striking part, Fig. 22 (b) is a rear view thereof, and Fig. 22 (c) is a cross-sectional view in a B-B direction.
Fig. 23 (a) shows a coupling relationship between the first inner case and the first striking part,
Fig. 23 (b) is a rear view thereof, and Fig. 23 (c) is a cross-sectional view in an F-F direction.
Fig. 24 shows a conventional method in which a mallet is used to manually apply an impact to a target object and insert it into a femur.

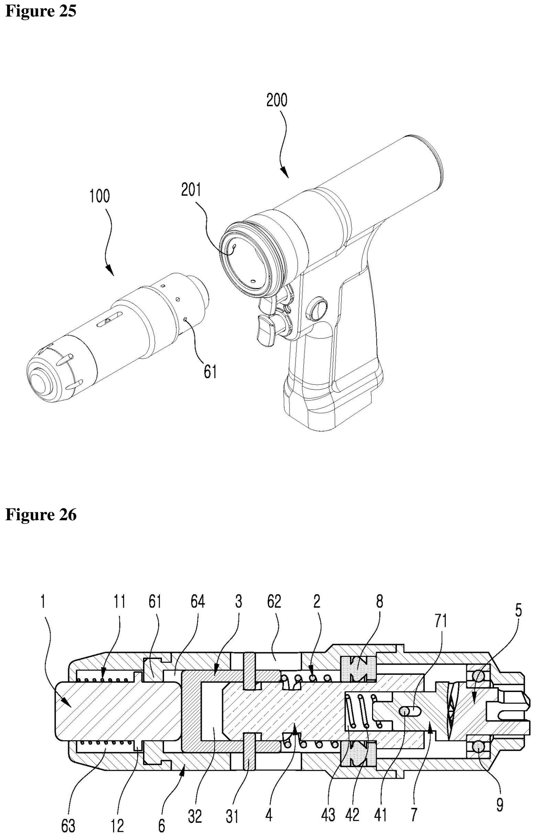

Fig. 25 and Fig. 26 show a conventional adapter.

### Best Mode for Carrying Out the Invention

Hereinafter, with reference to the accompanying drawings, embodiments of the present invention will be described in detail so that a person having ordinary knowledge in the technical field to which the present invention belongs can carry out the present invention. However, the present invention may be implemented in various different forms and is not limited to the embodiments described herein. Also, in the present embodiment, terms such as "first" and "second" are not used to indicate an order, but are terms used to distinguish components having the same name from each other.

The orthopedic adapter according to one embodiment of the present invention is an adapter detachably coupled to a rotary electric tool (e.g., a handpiece, a power tool).

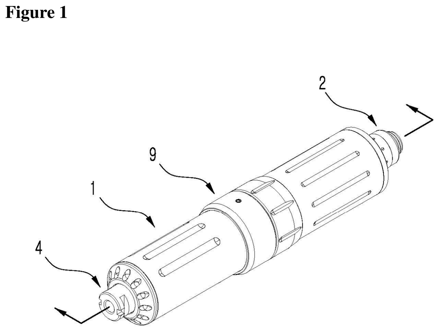

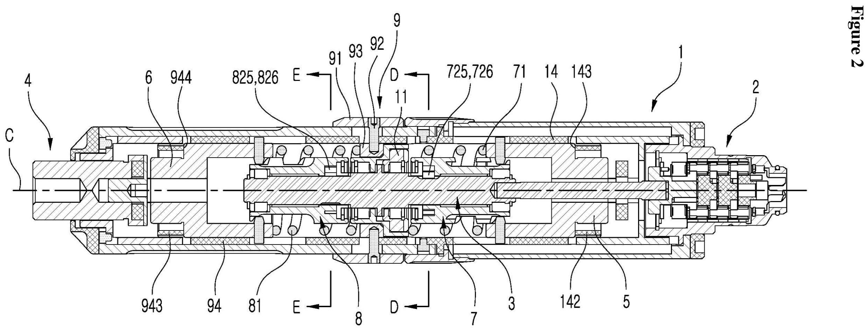

As shown in Fig. 1 and Fig. 2, the adapter of the present invention includes, as main components, a case part (1), a tool coupling part (2), a shaft (3), a first striking-part transfer part (7), a first striking part (5), a second striking-part transfer part (8), a second striking part (6), a force transmission part (4), and a force adjustment part (9).

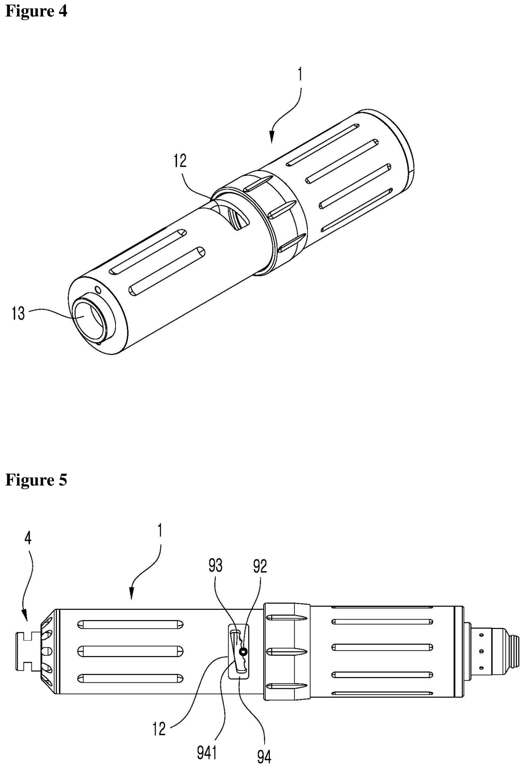

As shown in Fig. 1, Fig. 4, and Fig. 5, the case part (1) includes a circumferential through hole (12) through which a connection pin (92) to be described later can move, and a front opening (13) through which the force transmission part (4) protrudes.

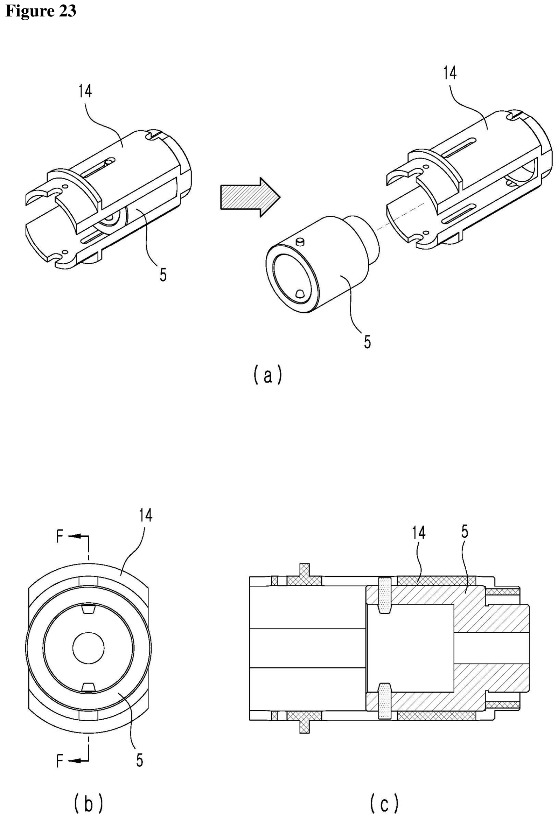

The tool coupling part (2) can rotate by receiving rotational force of a known rotary electric tool (e.g., a handpiece) (200) as shown in Fig. 23.

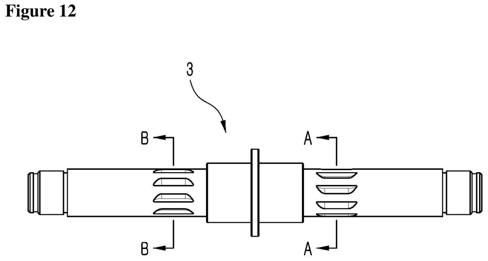

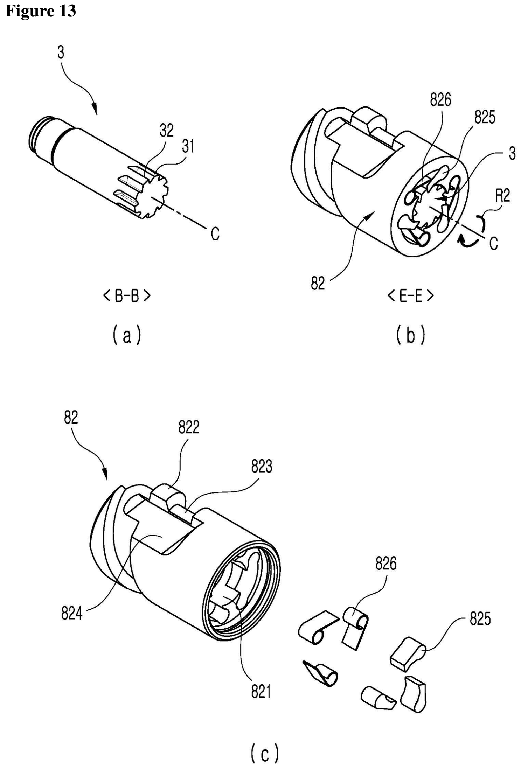

The shaft (3) rotates in interlock with a rotation direction of the tool coupling part (2). Also, as shown in Fig. 12 and Fig. 13, the shaft (3) has second shaft thread portions (31) and second shaft valley portions (32) alternately positioned in a circumferential direction thereof, and first shaft thread portions (33) and second shaft valley portions (34) alternately positioned in a circumferential direction thereof.

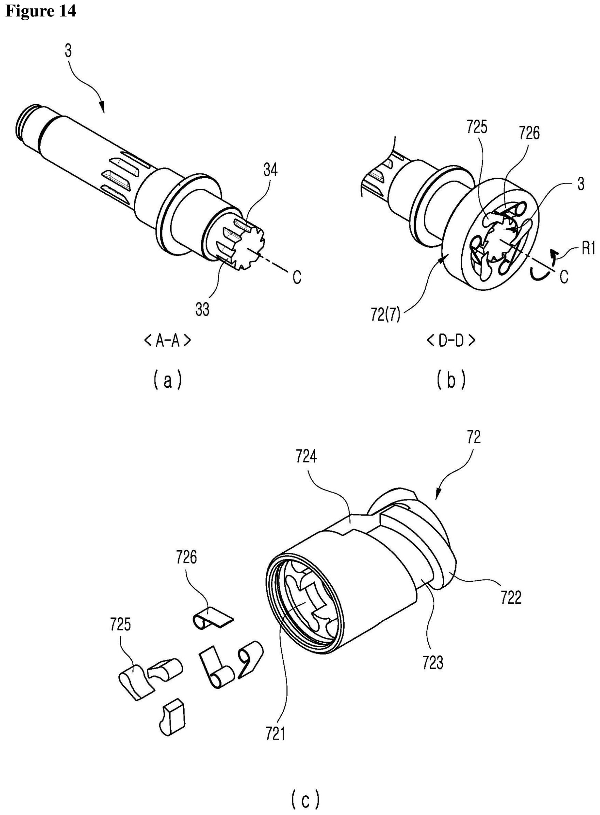

As shown in Fig. 14 (b), the first striking-part transfer part (7) rotates in interlock with the shaft (3) only when the shaft (3) rotates in a first direction (R1), for example, counterclockwise.

The first striking part (5) can move rearward (downward direction in Fig. 2, hereinafter the same) by rotation of the first striking-part transfer part (5).

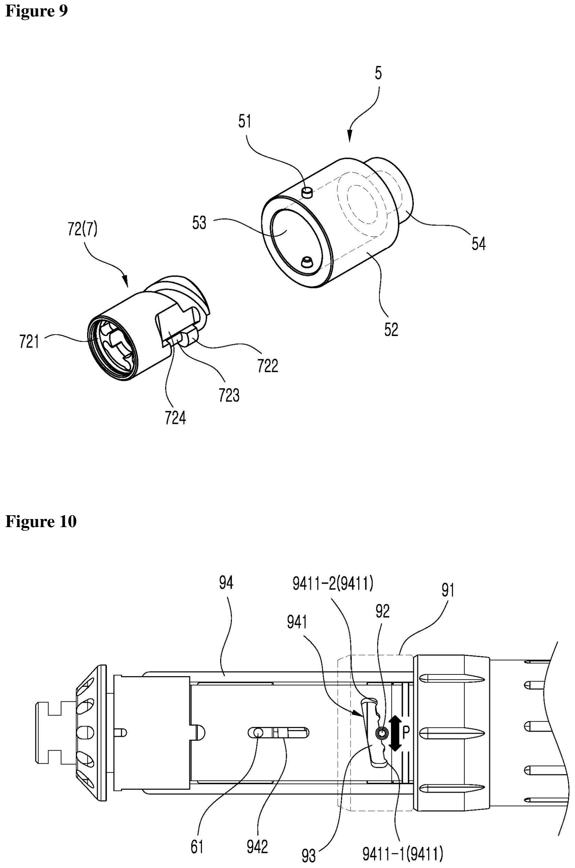

As shown in Fig. 9, the first striking part (5) has a first body part (52), a first bar (51), and a first protrusion part (54).

The first body part (52) has a first bore (bore) (53) penetrating the first body part (52) in a longitudinal direction.

The first bar (51) penetrates a wall of the first body part (52) so that one end protrudes into the first bore (53) and the other end protrudes to the outside of the first body part (52). Here, by way of example, two first bars (51) may be provided to face each other at 180° intervals in a circumferential direction of the first body part (52).

One end of the first bar (51) moves along the first thread valley portion (723) when the first body portion (72) rotates, and when the one end of the first bar (51) leaves the first thread valley portion (723) and reaches the first longitudinal groove portion (724), the first striking part (5) can move rearward by restoring force of the first elastic member (71).

Also, the first protrusion part (54) is connected to one end of the first body part (52) to form a step with the first body part (52), and has a bore connected to and communicating with the first bore (53).

As shown in Fig. 13 (b), the second striking-part transfer part (8) rotates in interlock with the shaft (3) only when the shaft (3) rotates in a second direction (R2), for example, clockwise.

The second striking part (6) can move forward (upward direction in Fig. 2, hereinafter the same) by rotation of the second striking-part transfer part (8).

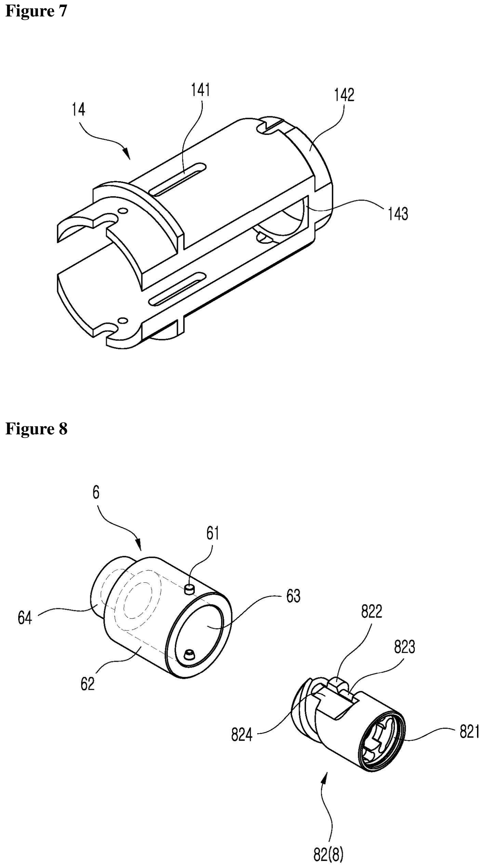

As shown in Fig. 8, the second striking part (6) has a second body part (62), a second bar (61), and a second protrusion part (64).

The second body part (62) has a second bore (bore) (63) penetrating the second body part (62) in a longitudinal direction.

The second protrusion part (64) is connected to one end of the second body part (62) to form a step with the second body part (62), and has a bore connected to and communicating with the second bore (63).

The second bar (61) penetrates a wall of the second body part (62) so that one end protrudes into the second bore (63) and the other end protrudes to the outside of the second body part (62). Here, by way of example, two second bars (61) may be provided to face each other at 180° intervals in a circumferential direction of the second body part (62).

One end of the second bar (61) moves along the second thread valley portion (823) when the second body part (62) rotates, and when the one end of the second bar (61) leaves the second thread valley portion (8723) and reaches the second longitudinal groove portion (824), the second striking part (6) can move forward by restoring force of the second elastic member (81).

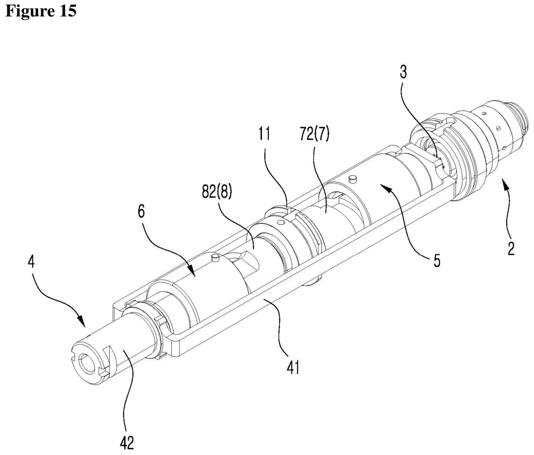

As shown in Fig. 15, the force transmission part (4) can move rearward by contact with the first striking part (5), and can move forward by contact with the second striking part (6).

The force transmission part (4) has a tool (e.g., a broach) connection part (42) protruding through the front opening (13) of the case part (1), and a frame (41) relatively slidable along the shaft (3).

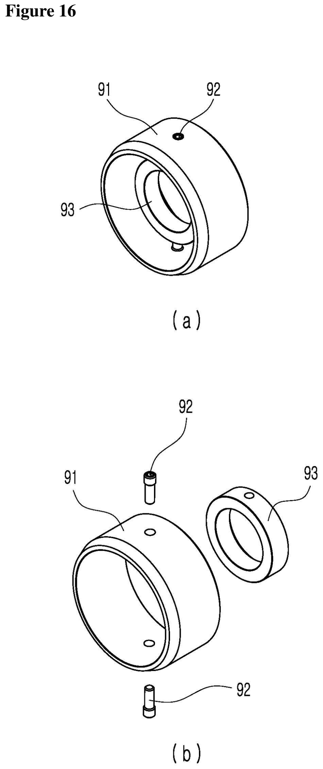

The force adjustment part (9) can adjust acceleration force by which the second striking part (6) moves forward, thereby adjusting a force that the second striking part (6) applies to the force transmission part (4). For this, as shown in Fig. 1, Fig. 5, Fig. 10, and Fig. 16, the force adjustment part (9) includes an outer ring (91), an inner ring (93), a connection pin (92), and a second inner case (94).

The outer ring (91) is mounted on the outside of the case part (1) to be rotatable relative to the case part (1).

The inner ring (93) is located inside the case part (1) and rotates in interlock with rotation of the outer ring (91).

The connection pin (92) connects the outer ring (91) and the inner ring (93) to each other and fixes them.

Referring to Fig. 1, Fig. 6, and Fig. 10, the second inner case (94) is fixed to the case part (1) between the outer ring (91) and the inner ring (93) inside the case part (1). Also, the second inner case (94) has a multi-stage pin hooking hole (941), and the multi-stage pin hooking hole

(941) has a plurality of hooking grooves (9411) through which the connection pin (92) passes and in which the connection pin (92) can be selectively caught, and is formed to be inclined to a central axis (C) of the shaft (3). Meanwhile, Fig. 10 shows main components of the force adjustment part (9) in a state where the outer ring is removed, and a multi-stage pin hooking hole and a connection pin may exist in the same manner also on the opposite side of the sheet of Fig. 10.

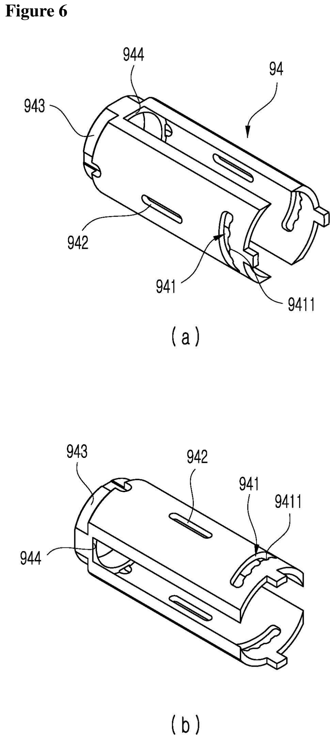

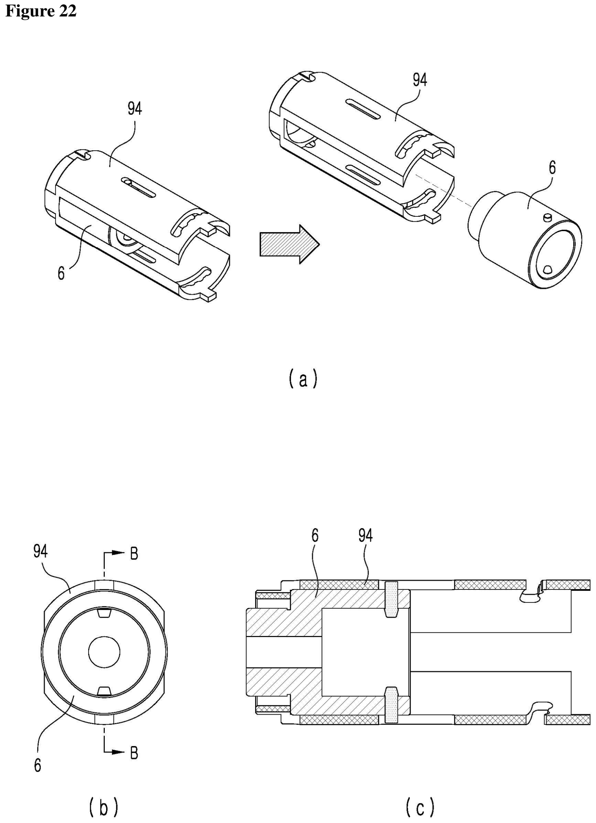

Also, as shown in Fig. 10, the second inner case (94) further has a second slit (942) through which the second bar (61) moves. And, as shown in Fig. 6 and Fig. 22, the second inner case (94) has a second abutment step (944). The second abutment step (944) functions as a stopper that can limit relative movement of the second body part (62) of the second striking part (6). For this, it has a second step part (943) having a smaller diameter than the remaining part of the second inner case (94).

As shown in Fig. 1 and Fig. 8 (b), the second striking-part transfer part (8) has a second elastic member (81) and a second body portion (82).

One end of the second elastic member (81) is supported by the inner ring (93), and the other end is supported by the second striking part (6).

The second body portion (82) has a second through hole (821) through which the shaft (3) passes, a plurality of second thread portions (822), a second thread valley portion (823) formed between the plurality of second thread portions (822), and a second longitudinal groove portion (824) extending in a longitudinal direction.

As shown in Fig. 1 and Fig. 9 (a), the first striking-part transfer part (7) has a first elastic member (71) and a first body portion (72).

One end of the first elastic member (71) is supported by a first spring support member (11) fixed to the case part (1), and the other end is supported by the first striking part (5).

The first body portion (72) has a first through hole (721) through which the shaft (3) passes, a plurality of first thread portions (722), a first thread valley portion (723) formed between the plurality of first thread portions (722), and a first longitudinal groove portion (724) extending in a longitudinal direction.

As shown in Fig. 13, the second body portion (82) has second ratchet parts (ratchet) (825, 826). For reference, a ratchet refers to a component that restricts movement of a machine element only in one direction.

The second ratchet parts (825, 826) have a second hook part (825) and a second pressing part (826) applying elastic force in a direction of always pressing the second hook part (825).

The second hook part (825) can be selectively caught in the second shaft valley portion (32), and when the shaft (3) rotates in the second direction (R2), for example, clockwise, does not disengage from the second shaft valley portion (32) so that the second body portion (82) can rotate in interlock with the shaft (3).

Meanwhile, when the shaft (3) rotates in the first direction (R1), for example, counterclockwise, the second hook part (825) overcomes elastic force of the second pressing part (826) by rotational force of the shaft (3) and moves sequentially to another neighboring second shaft valley portion (32) while rotating, whereby the second body portion (82) does not interlock with rotation of the shaft (3).

Also, as shown in Fig. 14, the first body portion (72) has first ratchet parts (725, 726).

The first ratchet parts (725, 726) have a first hook part (725) and a first pressing part (726) applying elastic force in a direction of always pressing the first hook part (725).

The first hook part (725) can be inserted into the first shaft valley portion (34), and when the shaft (3) rotates in the first direction (R1), does not disengage from the first shaft valley portion (34) so that the first body portion (72) can rotate in interlock with the shaft (3).

Meanwhile, when the shaft (3) rotates in the second direction (R2), the first hook part (725) overcomes elastic force of the first pressing part (726) by rotational force of the shaft (3) and moves sequentially to another neighboring first shaft valley portion (34) while rotating, whereby the first body portion (72) does not interlock with rotation of the shaft (3).

Meanwhile, as shown in Fig. 1 and Fig. 7, the first inner case (14) is fixed inside the case part (1) and surrounds the first striking part (5) and the first striking-part transfer part (7). Also, the first inner case (14) has a first slit (141) through which the first bar (51) moves. And, as shown in Fig. 9 and Fig. 23, the first inner case (14) has a first abutment step (143). The first abutment step (143) functions as a stopper that can limit relative movement of the first body part (52) of the first striking part (5). For this, it has a first step part (142) having a smaller diameter than the remaining part of the first inner case (14).

In addition, although not separately specified, in this embodiment, various known bearings may be appropriately provided at appropriate positions in order to reduce friction between components that rotate relative to each other.

Hereinafter, an operation of the impactor adapter according to one embodiment of the present invention having the above-described configuration will be described. For convenience of explanation, the rotary electric tool is not shown. Meanwhile, in the present embodiment, with respect to description of a configuration in which the second striking-part transfer part (8) moves the second striking part (6) forward by rotation of the shaft (3) and a configuration in which the first striking-part transfer part (7) moves the first striking part (5) rearward by rotation of the shaft (3), in order not to blur the point at issue, configurations different from those disclosed in Korean Registered Patent No. 2,343,886 of the present applicant will be mainly described, and description of the same configurations will be omitted or briefly made.

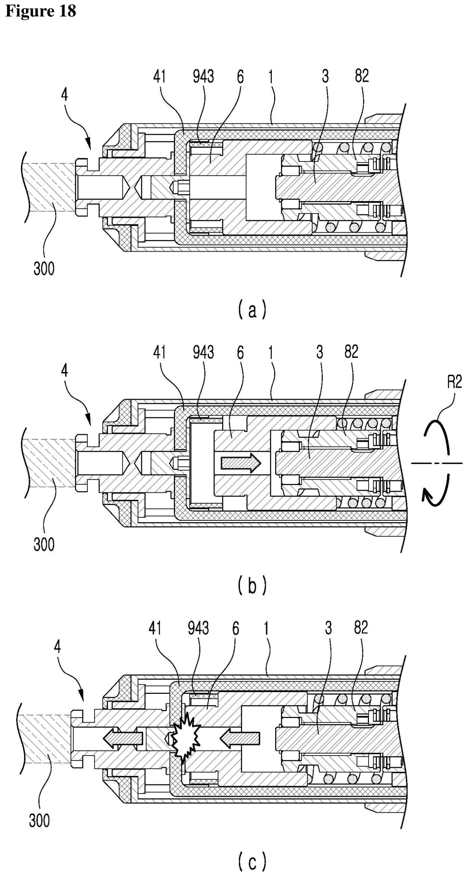

First, a process in which the adapter of the present invention strikes a surgical instrument, for example, a broach (300), forward in order to insert it will be described. At this time, by way of example, a user may perform striking while pressing the broach (300) with the adapter of the present invention. That is, as shown in Fig. 18 (a), by applying a force to the broach (300) with the adapter of the present invention in a state where the force transmission part (4) is in contact with the broach (300), the force transmission part (4) may move relatively to the right with respect to the case part (1), so that the frame (41) of the force transmission part (4) may be in a state of being in contact with the second step part (943).

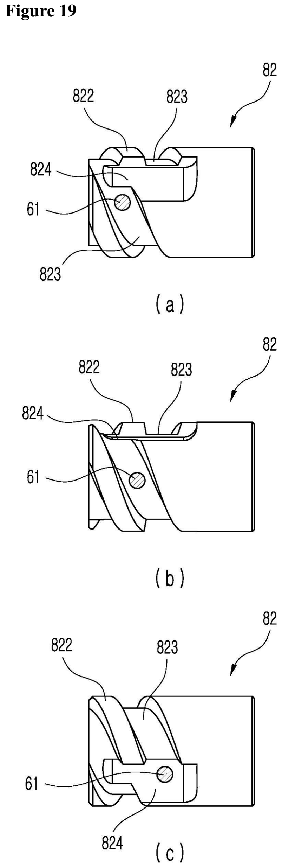

Fig. 18 (a) shows a state in which rotational force of a rotary electric tool (not shown) is not transmitted to the tool coupling part (2), that is, a non-operating state of the rotary electric tool. At this time, one end of the second bar (61) of the second striking part (6) is located in the thread valley portion (823) of the second body portion (82) (see Fig. 19 (a)).

Next, as shown in Fig. 13 and Fig. 18 (b), when rotational force in a second direction, for example, clockwise (R2), is applied to the shaft (3) by the rotary electric tool, the second body portion (82) also rotates in interlock with rotation of the shaft (3) by the second ratchet parts (825, 826). For reference, at this time, the first body portion (72) does not rotate in interlock with rotation of the shaft (3) due to the first ratchet parts (725, 726).

And as the second body portion (82) rotates, the second bar (61) of the second striking part (6) moves along the thread valley portion (823) of the second body portion (82). As the second striking part (6) gradually moves to the right, increasingly larger elastic energy is accumulated in the second spring (81) (see Fig. 19 (b)).

Next, as shown in Fig. 18 (c) and Fig. 19 (c), when the second bar (61) of the second striking part (6) leaves the second thread valley portion (823) of the body portion (82) of the second striking-part transfer part and reaches the second longitudinal groove portion (824), the second striking part (6) rapidly moves forward by restoring force of the second spring (81).

Finally, when the second striking part (6) strongly strikes the force transmission part (4), the force transmission part (4) transmits the striking force of the second striking part (6) to a target object (e.g., a broach). For reference, Fig. 19 (a) to Fig. 19 (c) are views respectively corresponding to Fig. 18 (a) to Fig. 18 (c), and schematically show relative positions of the second bar (61) in the second body portion (82) at each step.

Here, when the second body portion (82) rotates further, the second bar (62) of the second striking part (6) re-enters the second thread valley portion (823) of the second body portion (82), and repeats the process as described above.

Meanwhile, by using the force adjustment part (9), the force that the second striking part (6) applies to the force transmission part (4) can be adjusted as follows.

Referring to Fig. 10, by rotating the outer ring (91), an initial compression state of the second spring (81) can be adjusted.

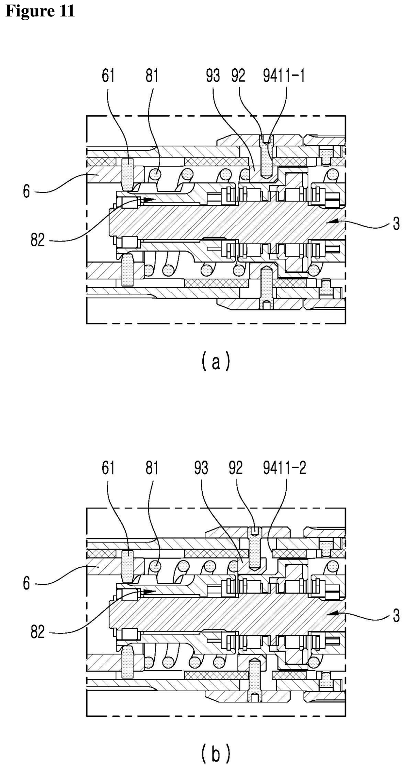

Specifically, when the outer ring (91) is rotated in an arrow (P) direction, the inner ring (93) also rotates together in interlock therewith by the connection pin (92). At this time, since the multi-stage pin hooking hole (914) is formed to be inclined to a central axis (C) of the shaft (3), for example, inclined so as to become closer to the force transmission part (4) as it goes upward (upward direction in Fig. 10), compression force of the second spring (81) can be adjusted depending on which hooking groove among the plurality of hooking grooves (9411) the connection pin (92) is located in. In Fig. 10, four hooking grooves (9411) are formed by way of example, and when located in the lowest hooking groove (9411-1), compression force of the second spring (81) becomes the smallest (see Fig. 11 (a)), and when located in the highest hooking groove (9411-2), compression force of the second spring (81) becomes the largest (see Fig. 11 (b)).

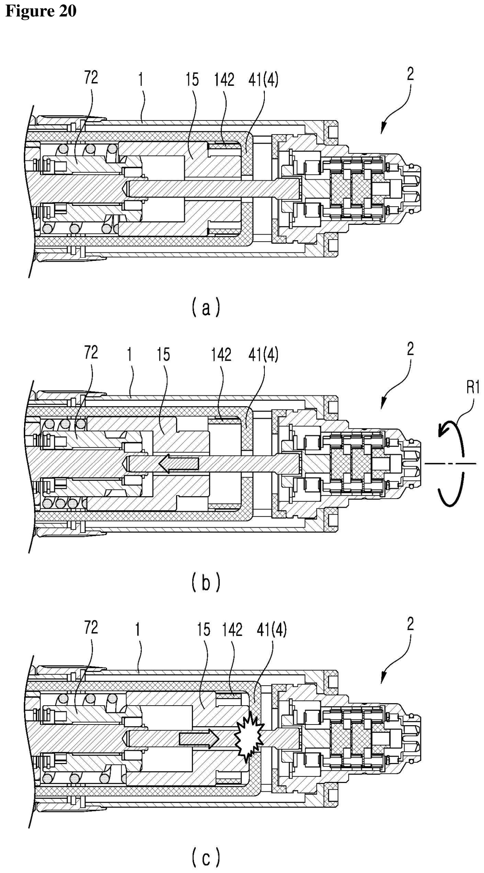

Hereinafter, an operation in which the adapter of the present invention applies a rearward (downward in Fig. 2) force to the force transmission part (4) in order to remove, for example, a broach inserted into a femur will be described. For reference, the broach is in a state of being connected to the force transmission part (4) directly or indirectly through a fastening means (not shown). By way of example, a user may perform rearward striking while pulling the broach (300) to the right (in Fig. 20) with the adapter of the present invention. At this time, as shown in Fig. 20 (a), the frame (41) of the force transmission part (4) may move relatively to the left with respect to the case part (1), so that the frame (41) of the force transmission part (4) may be in a state of being in contact with the first step part (142).

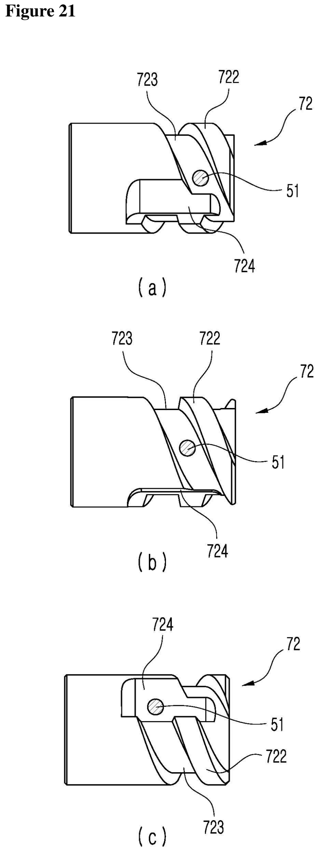

Fig. 20 (a) shows a state in which rotational force of the rotary electric tool is not transmitted to the tool coupling part (2), that is, a non-operating state of the rotary electric tool. At this time, one end of the first bar (51) of the first striking part (5) is located in the first thread valley portion (723) of the first body portion (72) (see Fig. 21 (a)).

Next, as shown in Fig. 14 and Fig. 20 (b), when rotational force in a first direction, for example, counterclockwise (R1), is applied to the shaft (3) by the tool coupling part (2), the first body portion (72) also rotates in interlock with rotation of the shaft (3) by the first ratchet parts (725, 726). For reference, at this time, the second body portion (82) does not rotate in interlock with rotation of the shaft (3) due to the second ratchet parts (825, 826).

And as the first body portion (72) rotates, the first bar (51) of the first striking part (5) moves along the first thread valley portion (723) of the first body portion (72). As the first striking part (5) gradually moves to the left (in Fig. 20), increasingly larger elastic energy is accumulated in the first spring (71) (see Fig. 20 (b) and Fig. 21 (b)).

Next, as shown in Fig. 20 (c), when the first bar (51) of the first striking part (5) leaves the first thread valley portion (723) of the first body portion (72) and reaches the first longitudinal groove portion (724), the first striking part (6) rapidly moves rearward (rightward in Fig. 20) by restoring force of the first spring (71).

Finally, when the first striking part (5) strongly strikes the force transmission part (4), that is, the frame (41) of the force transmission part, the force transmission part (4) applies a force in a direction of removing a target object (e.g., a broach). For reference, Fig. 21 (a) to Fig. 21 (c) are views respectively corresponding to Fig. 20 (a) to Fig. 20 (c), and schematically show relative positions of the first bar (51) in the first body portion (72) at each step.

Here, when the first body portion (72) rotates further, the first bar (51) of the first striking part (5) re-enters the first thread valley portion (723) of the first body portion (72), and repeats the process as described above.

Although preferred embodiments of the present invention have been described in detail above, the scope of rights of the present invention is not limited thereto, and various modifications and improvements by a person skilled in the art using the basic concept of the present invention defined in the following claims also belong to the scope of rights of the present invention.

### Industrial Applicability

The orthopedic adapter according to one embodiment of the present invention can be used in orthopedic surgery.

## Claims

1. An orthopedic adapter detachably coupled to a rotary electric tool, comprising:
a case part;
a tool coupling part receiving rotational force from the rotary electric tool;
a shaft rotating in interlock with a rotation direction of the tool coupling part;
a first striking-part transfer part rotating in interlock with the shaft only when the shaft rotates in a first direction;
a first striking part capable of moving rearward by rotation of the first striking-part transfer part;
a second striking-part transfer part rotating in interlock with the shaft only when the shaft rotates in a second direction;
a second striking part capable of moving forward by rotation of the second striking-part transfer part;
a force transmission part capable of moving rearward by contact with the first striking part and capable of moving forward by contact with the second striking part; and
a force adjustment part capable of adjusting a force that the second striking part applies to the force transmission part.

2. The orthopedic adapter of claim 1, wherein the force adjustment part comprises:
an outer ring mounted on an outside of the case part to be rotatable relative to the case part;
an inner ring located inside the case part and rotating in interlock with rotation of the outer ring;
a connection pin connecting the outer ring and the inner ring to each other; and
a second inner case fixed to the case part between the outer ring and the inner ring inside the case part, and having a multi-stage pin hooking hole through which the connection pin passes, in which a plurality of hooking grooves in which the connection pin can be selectively caught are formed, and which is formed to be inclined to a central axis of the shaft.

3. The orthopedic adapter of claim 2, wherein the second striking-part transfer part comprises:
a second elastic member of which one end is supported by the inner ring and the other end is supported by the second striking part; and
a second body portion having a second through hole through which the shaft passes, a plurality of second thread portions, a second thread valley portion formed between the plurality of second thread portions, and a second longitudinal groove portion extending in a longitudinal direction.

4. The orthopedic adapter of claim 1, wherein the first striking-part transfer part comprises:
a first elastic member of which one end is supported by a first spring support member fixed to the case part and the other end is supported by the first striking part; and
a first body portion having a first through hole through which the shaft passes, a plurality of first thread portions, a first thread valley portion formed between the plurality of first thread portions, and a first longitudinal groove portion extending in a longitudinal direction.

5. The orthopedic adapter of claim 3, wherein:
the shaft has second shaft thread portions and second shaft valley portions alternately positioned in a circumferential direction thereof;
the second body portion has second ratchet parts;
the second ratchet parts have a second hook part and a second pressing part applying elastic force in a direction of always pressing the second hook part; and
the second hook part can be caught in the second shaft valley portion and, when the shaft rotates in the second direction, does not disengage from the second shaft valley portion so that the second body portion can rotate in interlock with the shaft.

6. The orthopedic adapter of claim 5, wherein, when the shaft rotates in the first direction, the second hook part overcomes elastic force of the second pressing part by rotational force of the shaft and moves sequentially to another neighboring second shaft valley portion while rotating relative to the shaft, whereby the second body portion does not interlock with rotation of the shaft.

7. The orthopedic adapter of claim 4, wherein:
the shaft further has first shaft thread portions and second shaft valley portions alternately positioned in the circumferential direction thereof;
the first body portion has first ratchet parts;
the first ratchet parts have a first hook part and a first pressing part applying elastic force in a direction of always pressing the first hook part; and
the first hook part can be caught in the first shaft valley portion and, when the shaft rotates in the first direction, does not disengage from the first shaft valley portion so that the first body portion can rotate in interlock with the shaft.

8. The orthopedic adapter of claim 7, wherein, when the shaft rotates in the second direction, the first hook part overcomes elastic force of the first pressing part by rotational force of the shaft and moves sequentially to another neighboring first shaft valley portion while rotating relative to the shaft, whereby the first body portion does not interlock with rotation of the shaft.

9. The orthopedic adapter of claim 4, wherein the first striking part comprises:
a first body part in which a first bore is formed;
a first bar penetrating a wall of the first body part so that one end protrudes into the first bore and the other end protrudes to an outside of the first body part; and
a first protrusion part connected to one end of the first body part to form a step with the first body part,
wherein two first bars are provided to face each other at 180° intervals in a circumferential direction of the first body part.

10. The orthopedic adapter of claim 9, wherein one end of the first bar moves along the first thread valley portion when the first body portion rotates, and when the one end of the first bar leaves the first thread valley portion and reaches the first longitudinal groove portion, the first striking part moves rearward by restoring force of the first elastic member.

11. The orthopedic adapter of claim 3, wherein the second striking part comprises:
a second body part in which a second bore is formed;
a second bar penetrating a wall of the second body part so that one end protrudes into the second bore and the other end protrudes to an outside of the second body part; and
a second protrusion part connected to one end of the second body part to form a step with the second body part,
wherein two second bars are provided to face each other at 180° intervals in a circumferential direction of the second body part.

12. The orthopedic adapter of claim 11, wherein one end of the second bar moves along the second thread valley portion when the second body part rotates, and when the one end of the second bar leaves the second thread valley portion and reaches the second longitudinal groove portion, the second striking part moves forward by restoring force of the second elastic member.

13. The orthopedic adapter of claim 2, wherein the case part further comprises:
a circumferential through hole through which the connection pin can move; and
a front opening from which the force transmission part protrudes.

14. The orthopedic adapter of claim 13, wherein the force transmission part comprises:
a tool connection part protruding through the front opening of the case part; and
a frame relatively slidable along the shaft.

15. The orthopedic adapter of claim 11, wherein the second inner case further has a second slit through which the second bar moves.

16. The orthopedic adapter of claim 9, wherein a first inner case fixed inside the case part and surrounding the first striking part and the first striking-part transfer part is provided, and the first inner case has a first slit through which the first bar moves.
